# EUROPÄISCHE PATENTSCHRIFT

(11) **EP 3 365 053 B1**
(45) Veröffentlichungstag und Bekanntmachung des Hinweises auf die Patenterteilung: **19.06.2019**
(21) Anmeldenummer: 17790691.4
(22) Anmeldetag: 06.10.2017
(51) Int. Cl.: A61M 16/04, A61M 16/08

(54) **TRACHEAL-APPLIKATOR**
TRACHEAL APPLICATOR
APPLICATEUR TRACHÉAL

(30) Priorität: 04.11.2016 DE 102016013168
(43) Veröffentlichungstag der Anmeldung: 29.08.2018
(73) Patentinhaber: TNI medical AG, 97084 Würzburg (DE)
(72) Erfinder: KLAUS, Dieter, 79689 Maulburg (DE); ANGER, Ewald, 97246 Eibelstadt (DE); URBAN, Peter, 79102 Freiburg (DE); BREUTER, Christian, 97273 Kürnach (DE)
(74) Vertreter: advotec.
(86) Internationale Anmeldenummer: PCT/EP2017/075533
(87) Internationale Veröffentlichungsnummer: WO 2018/082864

(56) Entgegenhaltungen:
- EP-A1- 0 792 631
- WO-A1-2011/122964
- US-A- 5 582 161
- US-A1- 2009 139 530

## Beschreibung

Die vorliegende Erfindung bezieht sich auf das Gebiet der Atmungsunterstützungseinrichtungen, wie transnasale Insufflations- und Tracheotomiesysteme, hier speziell auf die Verbindung zwischen einem tracheotomierten Patienten und einem Highflow-Therapiegerät, wie es zur transnasalen Insufflation benutzt wird und mehrfach aus dem Stand der Technik bekannt ist (WO 2008/060 295 A2, DE 10 2006 019 402 A1). Im Nachfolgenden werden diese Geräte kurz als TNI-Geräte (transnasale Insufflationsgeräte) bezeichnet.

Die nasale Highflow Atemunterstützung mittels TNI-Gerät, auch nasale High-flow-Therapie (NHF) genannt, findet derzeit eine zunehmende Verbreitung in der Intensivmedizin, stellt sie doch nachgewiesener Weise eine gute Alternative zur bekannten Sauerstofftherapie und der Positivdruckbeatmung dar, die bei Patienten mit respiratorischer Insuffizienz eingesetzt werden. Zudem wird die nasale Highflow-Therapie als offenes System von den Patienten im Gegensatz zu den geschlossenen Drucksystemen mit fest sitzenden Atemmasken als angenehmer empfunden und findet auch von daher eine größere Akzeptanz.

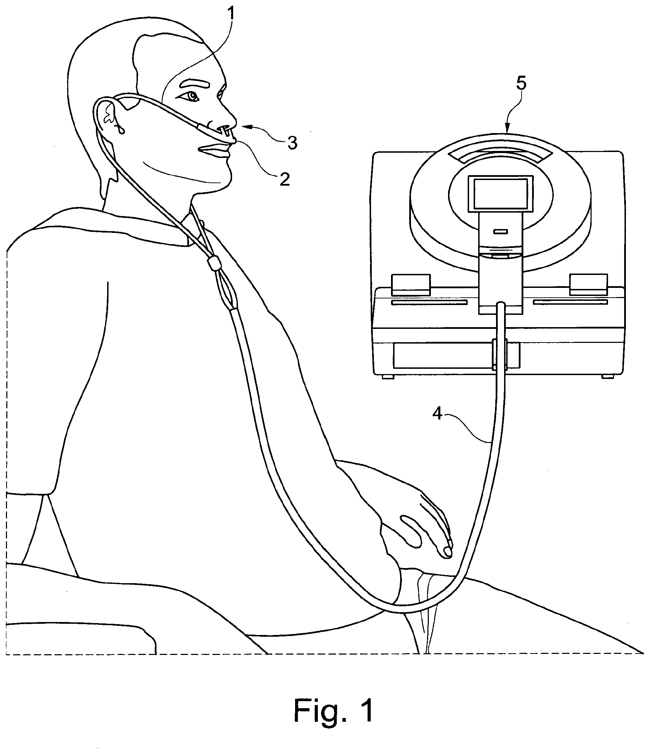

Die Therapie beruht auf der nasalen Applikation eines angewärmten und befeuchteten Luft/Sauerstoffgemisches FiO₂ mit einem Fluss von 2 bis 60 l/min, der von einem TNI-Gerät **(5)** kommend über einen Verbindungsschlauch **(4)** und Prongs **(2)** einer Nasenbrille **(1)** in die Nasenlöcher **(3)** appliziert wird (vgl. Fig. 1). Dabei ragen die Prongs **(2)** etwa 3 mm in die Nasenlöcher **(3)** hinein, wobei um die Prongs **(3)** herum genügend Raum bleibt, um in den Atempausen und während der Ausatmung das nicht benötigte Luft/Sauerstoffgemisch FiO₂ und die ausgeatmete verbrauchte Luft ins Freie abzublasen (offenes System). Aufgrund einer am TNI-Gerät **(5)** eingestellten Flussmenge, welche typisch größer ist als der Peak-Flow des Patienten, resultiert in der Nase ein stabiles Luft/Sauerstoffgemisch FiO₂ und es ergibt sich ein konstanter Versorgungszustand für den Patienten.

Bei der Applikation einer ausreichenden Flussmenge wird ein PEEP erzeugt, der sich über die Luftröhre bis in die alveoläre Ebene auswirkt und die Ventilation unterstützt. Dieser positive physikalische Effekt dient der Therapie im Besonderen. Nasensekrete und Feuchtigkeit können das offene System nicht beeinträchtigen, da sie ebenfalls an den Prongs **(2)** der Nasenbrille **(1)** vorbei ausgeschwemmt werden. Das warme und angefeuchtete Luft/Sauerstoffgemisch FiO₂ unterstützt dabei die mukoziliäre Reinigung.

Zusammengefasst ist die nasale Highflow-Therapie (NHF) eine neuartige, bereits erfolgreich eingesetzte Therapieform, mit der hohe Sauerstoffkonzentrationen angeboten werden können, eine Austrocknung der Atemwege vermieden wird, der Anteil der Totraumventilation reduziert und der nasale Totraum ausgewaschen wird. Weiterhin baut sich bei der Durchführung der Therapie ein endexpiratorischer positiver Atemwegsdruck auf, der einem Kollaps der Atemwege entgegenwirkt. Es ergibt sich eine Steigerung der Atmungseffizienz und eine Reduktion der benötigten Atmungsarbeit, was zur Entlastung der Atemmuskelpumpe beiträgt. All diese vorteilhaften Eigenschaften lassen den standardmäßigen Einsatz der NHF auch in Feldern der respiratorischen Insuffizienz, wie beispielsweise der chronischen Ateminsuffizienz, erwarten, wobei derzeit eine Reihe von Untersuchungen laufen und erste positive Ergebnisse vorliegen. Bei der NHF handelt es sich um eine nicht-invasive Beatmungsunterstützung, die meist nur zur intermittierenden Beatmung eingesetzt wird.

Anders verhält es sich bei der bekannten Beatmung über ein Tracheostoma, wobei die Patienten über eine Trachealkanüle, die die Halsweichteile durchstoßend in der Trachea (Luftröhre) liegt, beatmet werden. Diese, durch einen chirurgischen Eingriff ermöglichte invasive Beatmung wird meist als kontinuierliche Langzeitbeatmung zur Atemwegssicherung nach Unfällen und Operationen oder bei Weaning-Prozeduren eingesetzt, kann aber auch bis zu mehreren Stunden ausgesetzt werden, solange der tracheotomierte Patient dies duldet. Vorteile der Tracheometrie liegen u. a. darin, dass durch das Ausschalten der oberen Atemwege eine geringere Gefahr der Beschädigung der Stimmbänder besteht und einer Verringerung des Atemtotraumes, welche das Atmen für den Patienten erleichtert.

### Stand der Technik

Um die oben genannten Vorteile der NHF auch bei tracheotomierten Patienten nutzen zu können, wurden bereits Trachealkupplungen (Tracheal-Verbinder, -Interfaces oder -Applikatoren) entwickelt (US 5582161), die die auf dem Markt befindlichen Atemgasversorgungsgeräte, beispielsweise TNI-Geräte **(5)** bzw. deren beheizte Schlauchsysteme, unter Berücksichtigung der Aufrechterhaltung eines offenen Systems an die Trachealkanüle eines Tracheostoma ankoppeln.

So sind aus der DE 21 2011 100 071 U1 und der CA 2 843 533 A1 jeweils derartige Verbinder bekannt, die ein Highflow Atemgasversorgungssystem an einen Trachealeinsatz ankoppeln. Beide, aus diesen Druckschriften bekannte Verbinder weisen jeweils einen Einlassanschluss zum Aufnehmen eines einströmenden, von einer Gasquelle (z.B. TNI-Gerät) kommenden Gasflows (z.B. eines Luft/Sauerstoffgemisch FiO₂), einen Auslassanschluss als Ausatmungsport zur Bildung eines offenen Systems sowie einen sogenannten Patientenanschluss, der zur Verbindung mit einem Trachealeinsatz (z.B. einer Trachealkanüle) vorgesehen ist, auf. Bei beiden, aus diesen Druckschriften bekannten Trachealkupplungen ist der Einlassanschluss zwischen dem Patientenanschluss und dem Auslassanschluss angeordnet. Sowohl die Figuren 1, 3A bis 12 und 17 bis 28 der DE 21 2011 100 071 U1 als auch der darin in den Figuren 2A, 2B angegebene Stand der Technik und die Figuren 15 und 16 der CA 2 843 533 A1 zeigen jeweils einen seitlichen Einlassanschluss. Aus Letzterem ergeben sich konstruktionsbedingt im Inneren der Verbinder Turbulenzen (Wirbel), die zwar bei der Exspiration zur Flowdrosselung genutzt werden können, die aber den Ausfluss von Sekreten und Feuchtigkeit während der Exspirationphase oder beim Abhusten von Schleim negativ beeinträchtigen.

### Aufgabe und Lösung

Es ist daher Aufgabe der vorliegenden Erfindung, einen einfach aufgebauten Tracheal-Applikator zu schaffen, der die oben genannten Nachteil beseitigt. Die Lösung dieser Aufgabe gelingt durch eine idealisierte physikalische Nachbildung der Verhältnisse wie sie bei der Durchführung einer Highflow-Therapie am Nasenloch mit eingeführtem Prong vorhanden sind und erfindungsgemäß durch die im Patentanspruch 1 aufgeführten Merkmale spezifiziert sind. Weitere zweckmäßige oder vorteilhafte Ausgestaltungen des mit dem Patentanspruch 1 beanspruchten Tracheal-Applikators finden sich in den Unteransprüchen 2 bis 9. Atmungsunterstützungssysteme mit Tracheal-Applikatoren nach einem der Ansprüche 1 bis 9 sind Gegenstand des Verwendungsanspruchs 10.

### Vorteile der Erfindung

Der mit dem vorliegenden Patentanspruch 1 beanspruchte Tracheal-Applikator (6) zeichnet sich dadurch aus, dass er aufgrund seines speziellen Aufbaus unter Beibehaltung eines offenen Systems sowohl den Inspirationsfluss als auch den Exspirationsfluss annähernd turbulenz- und wirbelfrei leitet, dass in den Atempausen und während der Ausatmung das nicht benötigte Luft/Sauerstoffgemisch FiO₂ jederzeit ins Freie abgeblasen werden kann und Schleim- und Sekretbildungen jederzeit ausgeschwemmt werden können. Er ist aufgrund der beidseitig vorhandenen konisch zulaufenden Kopplungsmöglichkeiten darüber hinaus einfach und schnell austauschbar.

Das im Patentanspruch 2 angegebene kennzeichnende Merkmal sorgt in vorteilhafterweise für einen verbesserten Schleim- oder Sekretauswurf, da die an den Scheiteln der elliptischen Ausformung liegenden Entlüftungsöffnungen **(25)** größer sind und die Entlüftungsöffnung **(25),** die der Verengung **(11)** am nächsten liegt, aufgrund ihrer Schnaupen ähnlichen Form den Auswurf fördert.

Das kennzeichnende Merkmal des Patentanspruchs 3 vermeidet die Verstopfung einer Austrittsöffnung **(22)** durch Sekret- oder Schleimabsonderung, da die Ausflusswahrscheinlichkeit an der genannten Stelle bedingt durch die Schwerkraft am größten ist.

Die jeweiligen kennzeichnenden Merkmale der Patentansprüche 4, 5, 6 und 7 zeigen jeweils vorteilhafte Ausbildungen des Tracheal-Applikators **(6)** auf, die aufgrund ihrer Leitkörpereigenschaften und Form zur Vermeidung von Wirbeln- und Turbulenzen in den Gasströmen beitragen.

Das konstruktive kennzeichnende Merkmal des Patentanspruchs 8 bietet die vorteilhafte Voraussetzung, zwischen den im Patentanspruch 9 genannten Herstellungsarten wählen zu können. Im Bedarfsfalle ermöglicht die zylindrische Kontaktfläche **(16)** zudem in einfacher Weise einen zusätzlichen Anschlag zur Festlegung verschiedener Eindringtiefen des Verbindungsteils **(9)** in den Adapter **(8)** vorzusehen.

Der kennzeichnende Teil des Patentanspruchs 9 offenbart verschiedene Verbindungsmöglichkeiten zwischen Adapter **(8)** und Verbindungsteil **(9),** sodass unterschiedliche Herstellungsprozesse möglich sind.

Patentanspruch 10 zeigt eine vorteilbringende Verwendung des beanspruchten Tracheal-Applikators **(6)** in einem Atemunterstützungssystem auf, womit die Durchführung der bei der transnasalen Insufflation bereits bewährten Highflow-Therapien auch bei tracheotomierten Patienten ermöglicht wird.

### Ausführungsbeispiele

Ausgehend von einem in Figur 1 dargestellten Beatmungsunterstützungssystem nach dem Stand der Technik sind in den Figuren 2 bis 7 Ausführungsbeispiele der Erfindung, die weitere Vorteile und Besonderheiten offenbaren, dargestellt und werden im Folgenden näher beschrieben.

Es zeigen:
Fig. 1 ein nasales Highflow Atemunterstützungssystem mit TNI-Gerät und Nasenbrille gemäß dem Stand der Technik
Fig. 2 ein Highflow Atemunterstützungssystem mit TNI-Gerät und erfindungsgemäßem Tracheal-Applikator für tracheotomierte Patienten
Fig. 3 einen Tracheal-Applikator nach der Erfindung mit Anschlüssen an Trachealkanüle und Verbindungsschlauch zum TNI-Gerät in Explosionsdarstellung (3a) und im zusammengesteckten Zustand (3b)
Fig. 4 einen erfindungsgemäßen Tracheal-Applikator im Vollschnitt mit vergrößerter Detailzeichnung
Fig. 5 eine Schnittansicht B - B durch den Adapter nach Figur 4 mit vergrößerter Detailzeichnung
Fig. 6 einen Vollschnitt durch den Tracheal-Applikator nach Figur 4 mit eingezeichnetem Inspirationsfluss
Fig. 7 einen Vollschnitt durch den Tracheal-Applikator nach Figur 4 mit eingezeichnetem Expirationsfluss

Figur 1 zeigt ein nasales Highflow Atemunterstützungssystem mit einem TNI-Gerät **(5)** und einer Nasenbrille **(1)** nach dem Stand der Technik zur Durchführung einer nicht invasiven Beatmungsmethode. TNI-Gerät **(5)** und Nasenbrille **(1)** sind über einen beheizbaren Verbindungsschlauch **(4)** miteinander verbunden. Bei Anwendung der Highflow-Therapie (NHF) trägt der Patient eine Nasenbrille **(1)** mit Prongs **(2),** die etwa 3 mm in die Nasenlöcher **(3)** hineinragen. Um die Prongs **(2)** herum bleibt genügend Raum, um in den Atempausen während der Ausatmung (Expiration) das nicht benötigte Luft/Sauerstoffgemisch FiO₂ und die ausgeatmete verbrauchte Luft ins Freie abzublasen. Man spricht daher von einem "offenen System" mit dem Vorteil, dass Nasensekret und Feuchtigkeit ebenfalls an den Prongs **(2)** vorbei ausgeschwemmt werden können. Das TNI-Gerät **(5)** liefert die am Gerät eingestellte Flussmenge für die Einatmung (Inspiration). Therapiebedingt werden bei dieser Highflow-Applikation durch das TNI-Gerät **(5)** ein angewärmtes und befeuchtetes Luft/Sauerstoffgemisch FiO₂ mit einem Fluss bis etwa 60 l/min zugeführt. Die NHF Atmungsunterstützung führt nachweislich zu einer Verbesserung der Ventilation, insbesondere zur Reduktion des Anteils der Totraumventilation.

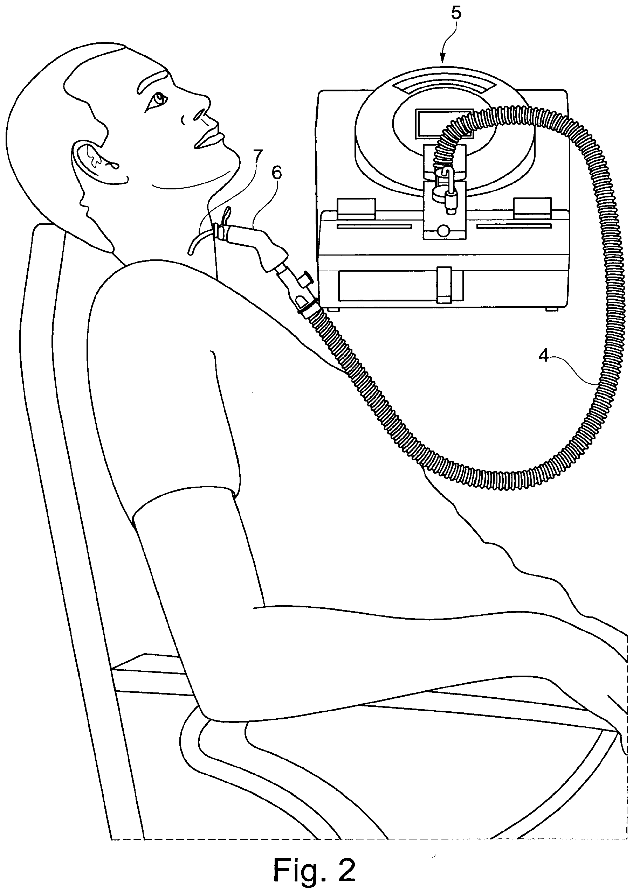

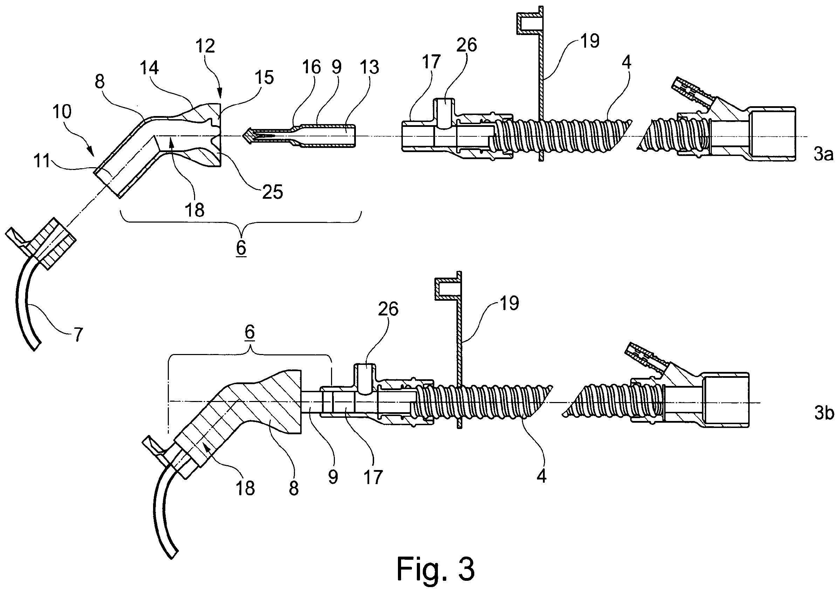

Figur 2 zeigt ein Highflow Atemunterstützungssystem mit einem TNI-Gerät **(5)** und einem erfindungsgemäßem Tracheal-Applikator **(6)** für tracheotomierte Patienten. Es ermöglicht die invasive Durchführung der bewährten Highflow Beatmung über die Trachealkanüle **(7)** eines Tracheostoma. Im Gegensatz zur nasalen Insufflation erfolgt hierbei die Einleitung hinter dem Oropharynx, sodass der Totraum in vorteilhafter Weise per se verkleinert ist. Das TNI-Gerät **(5)** ist dabei über einen beheizbaren Verbindungsschlauch **(4)** mit dem Tracheal-Applikator **(6)** verbunden, der seinerseits auf der Trachealkanüle **(7)** aufgesteckt ist. Der erfindungsgemäße Tracheal-Applikator **(6)** mit seinen in den Figuren 3 bis 5 näher gezeigten speziellen Aufbau ermöglicht die Verwendung des TNI-Gerätes **(5)** mit seinen bewährten Therapieeigenschaften bei einem tracheotomierten Patienten.

Figur 3 zeigt den Tracheal-Applikator **(6)** nach der Erfindung mit Anschlüssen an Trachealkanüle **(7)** und Verbindungsschlauch **(4)** zum TNI-Gerät in Explosionsdarstellung (3a) und im zusammengesteckten Zustand (3b).

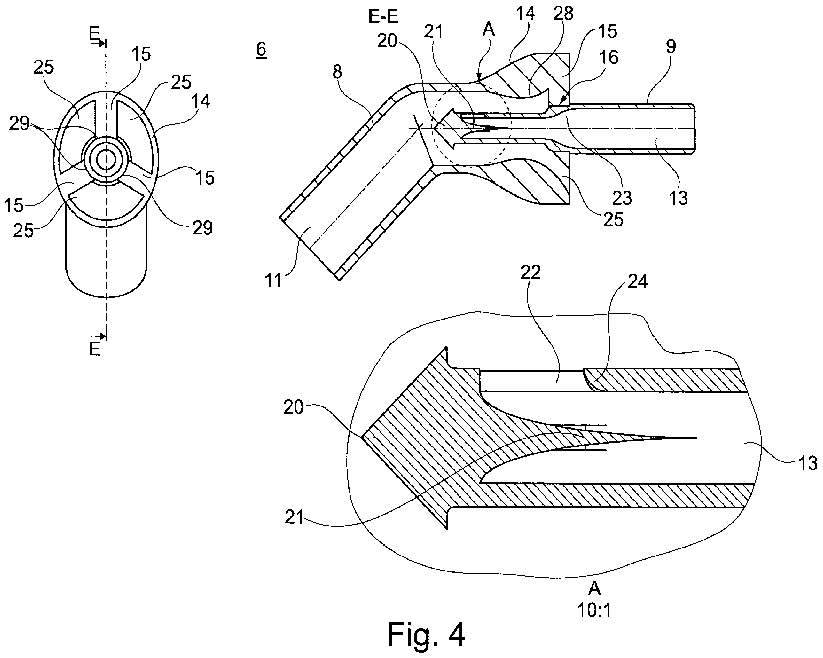

Figur 3a veranschaulicht, dass der Tracheal-Applikator **(6)** prinzipiell aus einem gekrümmten oder abgewinkelten rohrförmigen Adapter **(8)** und einem Verbindungsteil **(9)** besteht. Der Adapter **(8)** besitzt an seinem dem Tracheostoma zugewandten Ende **(10)** einen kreisförmigen Durchmesser mit einer konusförmigen Verengung **(11)** im Inneren zur steckbaren Verbindung mit der Trachealkanüle **(7).** Am anderen Ende **(12)** des Adapters **(8)** befindet sich das Verbindungsteil **(9),** das, wie hier gezeigt, in den Adapter **(8)** eingeschoben und mit ihm fest verbunden werden kann. Dazu weist das Verbindungsteil **(9)** die Form einer rotationssymmetrischen Röhre **(13)** mit einer zylindrischen Kontaktfläche **(16)** auf. Das Ende **(12)** des Adapters **(8)** ist als trompetenförmige Öffnung **(14)** ausgebildet, in der drei radiale Stege **(15)** (siehe auch Fig. 4) so nach innen ragen und geformt sind, dass sie eine Dreipunkt-Aufnahme **(29)** (vgl. Fig. 4) bilden, die das Verbindungsteil **(9)** beim Einschieben mittig aufnimmt und nach dem Einschieben bzw. Verpressen klemmend hält. Adapter **(8)** und Verbindungsteil **(9)** können alternativ auch verklebt, verschweißt oder bereits einstückig (z.B. im 3D-Druckverfahren) hergestellt sein. Die rotationssymmetrische Röhre **(13)** des Verbindungsteils **(9)** ist an ihrem Ende zum Verbindungsschlauch **(4)** hin ebenfalls so konisch gestaltet, dass sie an eine Schlauchverbindung **(17)** am Ende des Verbindungsschlauches **(4),** der zu einem TNI-Gerät führt, ankoppelbar ist. Das Bezugszeichen **(26)** an der Schlauchverbindung **(17)** bezieht sich auf einen Zugang für einen Temperaturmessfühler und das Bezugszeichen **(19)** auf eine Halterung für den beheizbaren Verbindungsschlauch **(4),** wie sie bei derartigen Systemen üblich sind.

In den Figuren 3a und 3b sind die Mittellinien der aufgezählten Teile gestrichelt eingezeichnet. Die Mittellinie **(18)** des Adapters **(8)** ist abgewinkelt oder abgeknickt dargestellt, um die Krümmung des Adapters **(8)** zu verdeutlichen. Die Krümmung ist notwendig, damit im zusammengesteckten oder -gefügten Zustand gemäß Fig. 3b die Trachealkanüle **(7)** möglichst frei von Kräften ist, die von dem Tracheal-Applikator **(6)** in Verbindung mit dem Verbindungsschlauch **(4)** ausgehen. Ein bevorzugter Knickwinkel der Mittellinie **(18)** liegt bei 60°, wobei je nach Person andere Knickwinkel besser geeignet und somit denkbar sein können. Unterstütz wird das Freihalten von auf die Trachealkanüle **(7)** wirkenden Kräften zusätzlich durch die am Verbindungschlauch **(4)** angeordnete Halterung **(19).**

Figur 4 zeigt einen erfindungsgemäßen Tracheal-Applikator **(6)** im Vollschnitt in einer Schnittansicht E - E, wie er im oberen linken Bild, das den Tracheal-Applikator **(6)** in einer Seitenansicht zeigt, eingezeichnet ist. Erkennbar ist, dass der Tracheal-Applikator **(6)** prinzipiell aus dem Adapter **(8)** und dem damit fest verbundenen Verbindungsteil **(9)** besteht. Der gekrümmte röhrenförmige Adapter **(8)** weist, wie bereits erwähnt, an einem Ende eine konusförmige, ins Innere ragende Verengung **(11)** zur steckbaren Verbindung mit einer Trachealkanüle **(7)** auf und ist an seinem entgegengesetzten Ende mit einer trompetenförmigen Öffnung **(14)** versehen, in der drei radial verlaufende Stege **(15)** so nach innen ragen und an ihrem Ende derart geformt sind, dass sie eine Dreipunkt-Aufnahme **(29)** zur festen Verbindung mit einer zylindrischen Kontaktfläche **(16)** des Verbindungsteils **(9)** bilden. Da die Stege **(15)** jeweils um 120° versetzt angeordnet sind, ergeben sich zwischen ihnen ausreichend große Entlüftungsöffnungen **(25)** zur Ausatmung des nicht benötigten Luft/Sauerstoffgemisches FiO₂ und der ausgeatmeten Luft sowie zur Ausschwemmung von Sekreten und Schleim zur Vermeidung von Stenosen. Das Verbindungsteil **(9),** das als düsenartig verengte rotationssymmetrische Röhre **(13)** zur Kanalisierung des von einem TNI-Gerät kommenden FiO₂- Flusses ausgestaltet ist, weist die zur Verbindung mit dem Adapter **(8)** notwendige zylindrische Kontaktfläche **(16)** auf und eine in den Adapter **(8)** hineinragende flaschenhalsförmig Verjüngung **(23)** zur Erhöhung der Strömungsgeschwindigkeit bei konstantem FiO₂-Fluss. Die im Bereich der Dreipunkt-Aufnahme **(16)** liegende flaschenhalsförmige Verjüngung **(23)** im Inneren der Röhre **(13)** hat absatzlose Übergänge ohne Stufen oder Kanten zur Aufrechterhaltung einer laminaren Strömung an dieser Stelle.

Die trompetenförmige Öffnung **(14)** ist durch eine Innenwölbung **(28)** so ausgeformt, dass bei der Inspiration die am TNI-Gerät **(5)** eingestellte, konstante Flussmenge nahezu wirbelfrei vom Verbindungsteil **(9)** durch den Adapter **(8)** in die Trachealkanüle **(7)** strömen kann.

Wie weiterhin aus Figur 4 ersichtlich, besitzt das in den Adapter **(8)** hineinragende Ende des Verbindungteiles **(9)** einen deckelförmigen Abschluss **(20),** der in Richtung Adapter **(8)** kegelförmig ist und in Richtung Schlauchverbindung **(17)** eine spitz zulaufende Kontur **(21)** aufweist, um als Strömungsleitkörper zu dienen. Wie in der Detailzeichnung A zu Figur 4 deutlicher gezeigt, kann diese Kontur **(21)** die Form eines Ellipsen- oder Hyperbelabschnitts annehmen. In der Röhre **(13)** sind in direkter Berührung mit dem Abschluss **(20)** mehrere radial verteilte und radial verlaufende Austrittsöffnungen **(22)** vorhanden, um in der Einatmungsphase das Luft/Sauerstoffgemisch FiO₂ aus dem Verbindungsteil **(9)** in den Adapter **(8)** zu leiten. Bevorzugt sind drei um 90° verteilte Austrittsöffnungen **(22)** vorhanden, wie insbesondere aus dem Schnittverlauf des gegebenen Beispiels in der Detailzeichnung A zu Figur 4 ersichtlich. Der Querschnitt der Kontur **(21)** als Strömungsleitkörper kann rund, quadratisch oder vieleckig sein, er richtet sich jeweils nach Anzahl und Anordnung der Austrittsöffnungen **(22).** Im angegebenen Beispiel mit drei Austrittsöffnungen **(22)** ist er rund. Erkennbar in dieser Detailzeichnung A ist auch eine Abrundung **(24)** in der Berandung einer der Austrittsöffnungen **(22)** der Röhre **(13),** diese sind bei allen Austrittsöffnungen **(22)** vorhanden, um die Strömung des Luft/Sauerstoffgemisches FiO₂ zu verbessern.

Der Rand der trompetenförmigen Öffnung **(14)** ist ellipsenförmig ausgeformt, wie aus der Seitenansicht des Tracheal-Applikators **(6)** auf der linken Seite in Figur 4 ersichtlich. Die große Achse der Ellipse verläuft durch einen der drei um 120° versetzt angeordneten Stege **(15)** und somit gleichzeitig durch die Mitte einer der drei Entlüftungsöffnungen **(25)** - im Weiteren "untere" Entlüftungsöffnung genannt -, wodurch deren dadurch bedingte Form die Sekret- und Schleimabsonderung begünstigt. Wie weiterhin aus Figur 4 erkennbar, ist im Bereich der unteren Entlüftungsöffnung **(25)** keine Austrittsöffnung **(22)** vorhanden, da an dieser Stelle eine Schleimabsonderung eine Austrittsöffnung **(22)** leicht verschließen könnte.

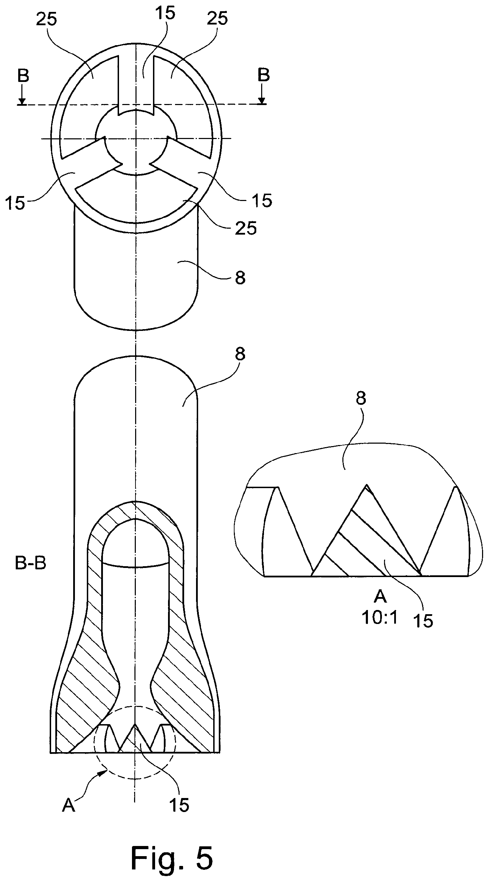

Figur 5 zeigt eine Schnittansicht B - B durch den Adapter **(8)** nach Figur 4 mit vergrößerter Detailzeichnung A, aus der die Querschnittsform der drei Stege **(15)** am besten ersichtlich ist. Die drei radial verlaufenden Stege **(15)** dienen nicht nur zur Verbindung des Adapters **(8)** mit dem Verbindungsteil **(9)** sondern bilden auch gleichzeitig die Trennwände zwischen den drei Entlüftungsöffnungen **(25),** wie bereits ausgeführt. Sie liegen damit als Strömungswiderstände voll im Exspirationsfluss und könnten durch Wirbelbildung das Austreten der Atemluft in die Umgebung behindern. Um die Strömungswiderstände klein zu halten und Wirbelbildungen im Inneren des Adapters **(8)** an diesen Stellen zu vermeiden, sind die Außenflächen der Stege **(15)** zum Inneren des Adapters **(8)** hin spitz zulaufend geformt, das bedeutet, sie bilden einen Spitzwinkel. Im dargestellten Ausführungsbeispiel hat der Querschnitt der Stege **(15)** die Form eines gleichseitigen Dreiecks. Es bieten sich aber auch andere Querschnitte an, z.B. in Form von spitzwinkligen, gleichschenkligen Dreiecken oder Tropfenformen mit Spitzwinkel.

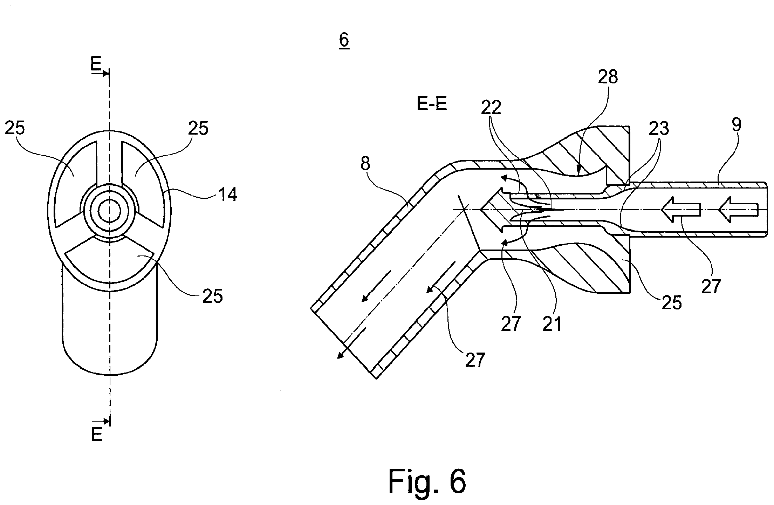

Figur 6 zeigt einen Vollschnitt durch den Tracheal-Applikator **(6)** nach Figur 4 mit eingezeichnetem Inspirationsfluss, repräsentiert durch die eingezeichneten Strömungspfeile **(27).** Der vom TNI-Gerät erzeugte und über den Verbindungsschlauch **(4)** und die Schlauchverbindung **(17)** in das Verbindungsteil **(9)** des Tracheal-Applikators **(6)** (vgl. Figur 3) gelangte FiO₂-Fluss durchströmt das Verbindungsteil **(9),** tritt dann durch die Austrittsöffnungen **(22)** hindurch in den Adapter **(8)** und gelangt von dort über die damit verbundene Trachealkanüle **(7)** (vgl. Figur 3) in die Lunge. Dies mit einem Druck, der 2 bis 3 mbar über dem äußeren Luftdruck liegt, um die Alveolen der Lunge ausreichend mit Atemluft zu versorgen. Da der Druck im Adapter **(8)** leicht höher ist, als der Luftdruck der Umgebungsluft, ist der Anteil der über die Entlüftungsöffnungen **(25)** miteingeatmeten Umgebungsluft sehr gering. Aufgrund der erfindungsgemäßen Anordnung von Verbindungsteil **(9)** mit Adapter **(8),** der einen Venturi-Effekt hervorrufenden flaschenhalsförmigen absatzlosen Verjüngung **(23),** der durch die Kontur **(21)** gegebenen Strömungsleitung vom Verbindungsteil **(9)** in den Adapter **(8)** sowie der Innenwölbung **(28)** in der trompetenförmigen Öffnung **(14)** des Adapters **(8)** ergibt sich für den Inspirationsfluss ein weitestgehend wirbelfreier Verlauf.

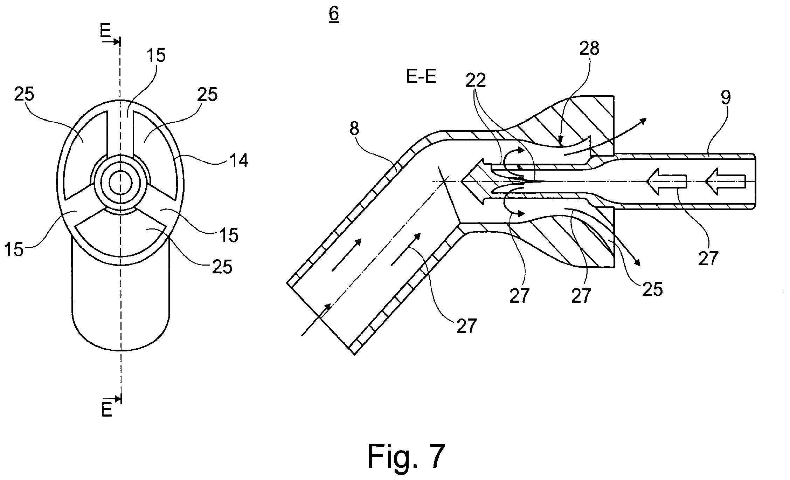

Figur 7 zeigt einen Vollschnitt durch den Tracheal-Applikator **(6)** nach Figur 4 mit eingezeichnetem Exspirationsfluss. Atmet der Patient über die Lunge aus, so liegt der Druck 4 bis 6 mbar über dem Druck während der Inspiration. Die verbrauchte und ausgeatmete Atemluft strömt durch die Trachealkanüle **(7)** in den Adapter **(8)** (vgl. Figur 3) und verlässt diesen durch die Entlüftungsöffnungen **(25)** der trompetenförmigen Öffnung **(14).** Da der vom TNI-Gerät erzeugte Druck des FiO₂₋Flusses in der Ausatmungsphase niedriger ist, als der Druck der ausgeatmeten Luft, strömt zwar weiterhin FiO₂-Fluss durch die Austrittsöffnungen **(22)** in den Adapter **(8),** wird dann aber von der ausgeatmeten Atemluft mitgenommen und durchströmt die Entlüftungsöffnungen **(25),** um folglich in die Umgebung abgegeben zu werden. Aufgrund des spitzwinkligen Profils der Stege **(15)** (vgl. Figur 5) kann hier der Exspirationsfluss wirbelfrei entweichen.

Der Tracheal-Applikator **(6)** nach der Erfindung kann aus einem beliebigen medizinischen Kunststoff hergestellt oder mit einem antibakteriellen polymeren Überzug (coating) versehen sein.

### Bezugszeichenliste

- 1: Nasenbrille
- 2: Prongs
- 3: Nasenlöcher
- 4: Verbindungsschlauch
- 5: TNI-Gerät / Highflow-Therapiegerät
- 6: Tracheal-Applikator
- 7: Trachealkanüle
- 8: Adapter
- 9: Verbindungsteil
- 10: Ende
- 11: konusförmige Verengung
- 12: Ende
- 13: rotationssymmetrische Röhre
- 14: trompetenförmige Öffnung
- 15: Stege
- 16: zylindrische Kontaktfläche
- 17: Schlauchverbindung
- 18: Mittellinie
- 19: Halterung
- 20: Abschluss
- 21: Kontur
- 22: Austrittsöffnung
- 23: flaschenförmige Verjüngung
- 24: Abrundungen
- 25: Entlüftungsöffnungen
- 26: Zugang für Temperaturfühler
- 27: Strömungspfeile
- 28: Innenwölbung
- 29: Dreipunkt-Aufnahme

## Patentansprüche

1. Tracheal-Applikator (6),
• der aus einem gekrümmten, rohrförmigen Adapter (8) mit kreisförmigem Durchmesser besteht, der an einem Ende eine konusförmige, ins Innere ragende Verengung (11) zur steckbaren Verbindung mit einer Trachealkanüle (7) aufweist und am anderen Ende (12) mit einer trompetenförmigen Öffnung (14) versehen ist, und ein Verbindungsteil (9) aufweist, wobei in der trompetenförmigen Öffnung (14) drei radial verlaufende Stege (15) ins Innere des Adapters ragen, um als Dreipunkt-Aufnahme (29) zur mittigen Halterung und Befestigung des Verbindungsteils (9) des Tracheal-Applikators (6) zu dienen und um gleichzeitig drei Entlüftungsöffnungen (25) zu bilden, und wobei
• das Verbindungsteil (9) aus einer düsenartig verengten, rotationssymmetrischen Röhre (13) zur Kanalisierung eines von einem Highflow-Therapiegerät (5) kommenden FiO₂ Flusses besteht, dessen in den Adapter (8) hineinragender Teil eine flaschenhalsförmige Verjüngung (23) besitzt, am verjüngten Ende mit einem Abschluss (20) versehen ist und in direkter Berührung mit diesem Abschluss (20) mehrere radial verteilte und radial verlaufende Austrittsöffnungen (22) aufweist, und wobei das andere Ende des Verbindungsteils (9) konisch zulaufend geformt ist, um mit einem Verbindungsschlauch (4) eines Highflow-Therapiegerätes (5) verpressbar gekoppelt zu werden.

2. Tracheal-Applikator (6) nach Patentanspruch 1,
**dadurch gekennzeichnet, dass**
• der Rand der trompetenförmigen Öffnung (14) ellipsenförmig ausgeformt ist.

3. Tracheal-Applikator (6) nach Patentanspruch 2,
**dadurch gekennzeichnet, dass**
• innerhalb des Adapters (8) keine Austrittsöffnung (22) vorhanden ist, die parallel zur längeren Symmetrieachse der elliptischen Ausformung der trompetenförmigen Öffnung (14) verläuft und in Richtung desjenigen Scheitels der elliptischen Ausformung zeigt, der der Verengung (11) am nächsten liegt.

4. Tracheal-Applikator (6) nach einem der Patentansprüche 1 bis 3,
**dadurch gekennzeichnet, dass**
• die Austrittsöffnungen (22) Abrundungen (24) in ihrer Berandung aufweisen.

5. Tracheal-Applikator (6) nach einem der Patentansprüche 1 bis 4,
**dadurch gekennzeichnet, dass**
• der Abschluss (20) in Richtung des Adapters (8) kegelförmig geformt ist und in Richtung einer Schlauchverbindung (17) eine spitz zulaufende Kontur (21) aufweist, die ins Innere des Verbindungsteils (9) ragt und als Strömungsleitkörper dient.

6. Tracheal-Applikator (6) nach einem der Patentansprüche 1 bis 5,
**dadurch gekennzeichnet, dass**
• die Außenflächen der Stege (15) zum Inneren des Adapters (8) hin spitz zulaufend geformt sind.

7. Tracheal-Applikator (6) nach einem der Patentansprüche 1 bis 6,
**dadurch gekennzeichnet, dass**
• die trompetenförmige Öffnung (14) eine Innenwölbung (28) aufweist.

8. Tracheal-Applikator (6) nach einem der Patentansprüche 1 bis 7,
**dadurch gekennzeichnet, dass**
• das Verbindungsteil (9) eine zylindrische Kontaktfläche (16) aufweist, die zur Verbindung mit der Dreipunkt-Aufnahme (29) des Adapters (8) dient.

9. Tracheal-Applikator (6) nach Patentanspruch 8,
**dadurch gekennzeichnet, dass**
• Adapter (8) und Verbindungsteil (9) an den Berührungspunkten zwischen der Dreipunkt-Aufnahme (29) und der zylindrischen Kontaktfläche (16) verklemmt, verklebt oder verschweißt sind oder der Tracheal-Applikator einstückig hergestellt ist.

10. Tracheales Atemunterstützungssystem mit einem Highflow-Therapiegerät (5) mit Verbindungsschlauch (4) und einem Tracheostoma mit Trachealkanüle (7), **gekennzeichnet durch** die Verwendung eines sie verbindenden Tracheal-Applikators (6) nach einem der Ansprüche 1 bis 9.

## Claims

1. A tracheal applicator (6),
said applicator (6) comprising a curved tube-shaped adapter (8) having a circular diameter which at one end comprises a conical constriction (11), which protrudes into the interior, for a plug-in connection with a tracheal cannula (7) and at the other end (12) is provided with a trumpet-shaped opening (14) and comprises a connecting piece (9), three radially extending crosspieces (15) protruding into the interior of the adapter in the trumpet-shaped opening (14) in order to serve as a three-point reception (29) for centrally holding and securing the connecting piece (9) of the tracheal applicator (6) and to simultaneously form three ventilation openings (25),
and the connecting piece (9) comprising a rotationally symmetric tube (13) constricted in a nozzle-like manner for channeling a FiO₂ flow coming from a high flow therapy device (5), the part of the connecting piece (9) protruding into the adapter (8) comprising a bottleneck-shaped tapering (23) and being provided with a seal (20) at the tapered end and comprising several radially distributed and radially extending outlet openings (22) in direct contact with said seal (20), and the other end of the connecting piece (9) being designed so as to be conically tapering in order to be coupled to a connecting tube (4) of a high flow therapy device (5) by pressing.

2. The tracheal applicator (6) according to claim 1,
**characterized in that**
the edge of the trumpet-shaped opening (14) is formed elliptical.

3. The tracheal applicator (6) according to claim 2,
**characterized in that**
within the adapter (8), no outlet opening (22) is provided which extends parallel to the longer symmetric axis of the elliptical shape of the trumpet-shaped opening (14) and points in the direction of the apex of the elliptical shape closest to the constriction (11).

4. The tracheal applicator (6) according to any one of the claims 1 to 3,
**characterized in that**
the outlet openings (22) have roundings (24) at their edges.

5. The tracheal applicator (6) according to any one of the claims 1 to 4,
**characterized in that**
the seal (20) is formed conical in the direction of the adapter (8) and comprises a tapered contour (21) in the direction of a tube connection (17), said contour (21) protruding into the interior of the connecting piece (9) and serving as a flow conducting body.

6. The tracheal applicator (6) according to any one of the claims 1 to 5,
**characterized in that**
the outer surfaces of the crosspieces (15) are formed tapering towards the interior of the adapter (8).

7. The tracheal applicator (6) according to any one of the claims 1 to 6,
**characterized in that**
the trumpet-shaped opening (14) has an inner bulge (28).

8. The tracheal applicator (6) according to any one of the claims 1 to 7,
**characterized in that**
the connecting piece (9) comprises a cylindrical contact surface (16) which serves for being connected to the three-point reception (29) of the adapter (8).

9. The tracheal applicator (6) according to claim 8,
**characterized in that**
the adapter (8) and the connecting piece (9) are connected at the contact points between the three-point reception (29) and the cylindrical contact surface (16) by clamping, by adhesion or by welding or **in that** the tracheal applicator is produced in one piece.

10. A tracheal ventilatory support system comprising a high flow therapy device (5) comprising a connecting tube (4) and a tracheostoma comprising a tracheal cannula (7), **characterized by** the use of a tracheal applicator (6), which connects the high flow therapy device and the tracheostoma, according to any one of the claims 1 to 9.

## Revendications

1. Applicateur trachéal (6),
ledit applicateur (6) comprenant un adaptateur (8) courbé, en forme de tuyau et ayant un diamètre circulaire, ledit adaptateur ayant à une extrémité une constriction (11) conique, qui se projette dans l'intérieur, pour réaliser une connexion enfichable avec une canule trachéale (7), et à l'autre extrémité (12) étant pourvu d'une ouverture (14) en forme de trompette et comprenant une pièce de liaison (9), trois traverses (15) s'étendant radialement se projetant dans l'intérieur de l'adaptateur dans l'ouverture (14) en forme de trompette afin de servir en tant que logement trois points (29) pour tenir et fixer centralement la pièce de liaison (9) de l'applicateur trachéal (6) et pour former en même temps trois ouvertures de ventilation (25),
et la pièce de liaison (9) étant formée par un tuyau (13) symétrique en rotation, rétréci à la manière d'une buse et servant à canaliser un écoulement FiO₂ venant d'un dispositif de thérapie High-Flow (5), la partie de ladite pièce de liaison (9) se projetant à l'intérieur de l'adaptateur (8) comprenant un effilement (23) en forme de goulot et étant pourvue d'un joint (20) à l'extrémité effilée et comprenant plusieurs ouvertures de sortie (22) distribuées radialement et s'étendant radialement en contact direct avec ledit joint (20), et l'autre extrémité de la pièce de liaison (9) étant réalisée de sorte à être effilée en forme conique afin d'être reliée à un tuyau de liaison (4) d'un dispositif de thérapie High-Flow (5) par compression.

2. Applicateur trachéal (6) selon la revendication 1,
**caractérisé en ce que**
le bord de l'ouverture (14) en forme de trompette a une forme elliptique.

3. Applicateur trachéal (6) selon la revendication 2,
**caractérisé en ce que**
à l'intérieur de l'adaptateur (8), aucune ouverture de sortie (22) est prévue qui s'étend parallèlement à l'axe symétrique le plus long de la forme elliptique de l'ouverture (14) en forme de trompette et est orientée dans le sens du sommet de la forme elliptique qui est le plus proche de la constriction (11).

4. Applicateur trachéal (6) selon l'une quelconque des revendications 1 à 3,
**caractérisé en ce que**
les ouvertures de sortie (22) ont des courbatures (24) à leurs bords.

5. Applicateur trachéal (6) selon l'une quelconque des revendications 1 à 4,
**caractérisé en ce que**
le joint (20) est réalisé en forme conique dans le sens de l'adaptateur (8) et comprend un contour (21) effilé en pointe dans le sens d'une connexion de tuyau (17), ledit contour (21) se projetant dans l'intérieur de la pièce de liaison (9) et servant en tant que corps de guidage d'écoulement.

6. Applicateur trachéal (6) selon l'une quelconque des revendications 1 à 5,
**caractérisé en ce que**
les surfaces extérieures des traverses (15) sont formées de telle manière qu'elles sont effilées en pointe vers l'intérieur de l'adaptateur (8).

7. Applicateur trachéal (6) selon l'une quelconque des revendications 1 à 6,
**caractérisé en ce que**
l'ouverture (14) en forme de trompette a un renflement intérieur (28).

8. Applicateur trachéal (6) selon l'une quelconque des revendications 1 à 7,
**caractérisé en ce que**
la pièce de liaison (9) comprend une surface de contact (16) cylindrique qui sert à être reliée au logement trois points (29) de l'adaptateur (8).

9. Applicateur trachéal (6) selon la revendication 8,
**caractérisé en ce que**
l'adaptateur (8) et la pièce de liaison (9) sont reliés aux points de contact entre le logement trois points (29) et la surface de contact (16) cylindrique par coincement, par adhésion ou par soudage ou **en ce que** l'applicateur trachéal est produit en une seule pièce.

10. Système de support de ventilation trachéal qui comprend un dispositif de thérapie High-Flow (5) comprenant un tuyau de liaison (4) et une trachéostomie comprenant une canule trachéale (7), **caractérisé par** l'utilisation d'un applicateur trachéal (6), qui relie le dispositif de thérapie High-Flow et la trachéostomie, selon l'une quelconque des revendications 1 à 9.
